# EUROPEAN PATENT APPLICATION

(11) **EP 2 455 021 A2**
(43) Date of publication of application: **23.05.2012**
(21) Application number: 11250755.3
(22) Date of filing: 09.09.2011
(51) Int. Cl.: A61B 17/34, A61M 13/00

(54) **Access apparatus including desufflation control mechanism**

(30) Priority: 23.11.2010 US 416559 P; 28.07.2011 US 192712
(71) Applicant: Tyco Healthcare Group LP, New Haven, CT 06511 (US)
(72) Inventor: Hartoumbekis, Elias, New Haven, CT 06512 (US); Richard, Paul D., Shelton, CT 06484 (US)
(74) Representative: Soames, Candida Jane

(57) **Abstract**

An access apparatus for use in surgical procedures is provided. The access apparatus includes a control mechanism that is configured to control desufflation flow of the access apparatus. The control mechanism includes a valve in fluid communication with a longitudinal passage that is operably associated with the access apparatus. The valve includes one or more openings and a selectively movable mechanical interface that operably couples to the valve. The selectively movable mechanical interface is configured to alter desufflation flow when it is moved with respect to the valve and across the at least one opening.

## Description

### CROSS REFERENCE TO RELATED APPLICATION

The present application claims the benefit of and priority to U.S. Provisional Application Serial No. 61/416,559 filed on November 23, 2010, the entire contents of which are incorporated herein by reference.

### BACKGROUND

### Technical Field

The present disclosure relates to an access apparatus and, more particularly, to an access apparatus that includes a desufflation control mechanism.

### Description of Related Art

In laparoscopic procedures, clinicians perform surgery in the interior of the abdomen through a small incision, and in endoscopic procedures, clinicians conduct surgery in any hollow viscus of the body through a narrow tube or cannula inserted through a small entrance incision in the skin. In certain instances, one or more insufflation ports are operably associated with the narrow tube or cannula and are configured to provide a pressurized gas, e.g., CO₂, into the abdominal cavity after the narrow tube or cannula is inserted into the incision and secured to a patient, thus creating a pneumoperitoneum. The pressurized gas provides a positive pressure that raises the inner body wall away from internal organs, thereby providing the surgeon with an operating space. By creating an operating space, the clinician avoids unnecessarily contacting the organs with the instruments inserted through the cannula assembly.

During the surgical procedure it may prove necessary for a clinician to alter the amount of pressurized gas in the abdominal cavity. That is, during a surgical procedure it may prove necessary, under certain surgical environments, to further insufflate and/or desufflate the abdominal cavity. For example, during one of the previously described surgical procedure, e.g., a laparoscopic procedure, where tissue may be ligated and/or cauterized, plumes of smoke may fill the abdominal cavity. As can be appreciated, a smoke filled abdominal cavity is not typically desired by a clinician. More particularly, the smoke contained within the abdominal cavity may, *inter alia,* prevent a clinician from clearly viewing the tissue being treated. Typically, clearing and/or evacuating the smoke contained in the abdominal cavity is achieved by desufflating the abdominal cavity. Under certain surgical environments, it may prove necessary to increase desufflation flow to accelerate the clearing and/or evacuating of the smoke from the abdominal cavity.

Accordingly, it may prove advantageous for a clinician to have the capability to more effectively control desufflation flow of the pressurized gas within the abdominal cavity such that the clinician may effectively treat tissue within the abdominal cavity.

### SUMMARY

The present disclosure provides an access apparatus for use in surgical procedures. The access apparatus includes an access member that defines a longitudinal axis and has a longitudinal passage. The longitudinal passage adapted to permit passage a surgical instrument utilized in performing a surgical procedure. A housing includes a proximal end that defines an opening in communication with the longitudinal passage of the access member to permit passage of the surgical instrument. A zero-closure valve is disposed within the longitudinal passage and is configured to provide a substantially fluid-tight seal in the absence of the surgical instrument inserted therethrough. A control mechanism operably coupled to the access apparatus is configured to control desufflation flow of the access apparatus. The control mechanism includes an elongated valve that is in fluid communication with the longitudinal passage. The valve includes one or more openings that are operably disposed along a length thereof. The control mechanism includes a selectively movable mechanical interface that is operably coupled to the valve and in operative communication with the opening. The selectively movable mechanical interface is configured to alter desufflation flow when it is moved along a length of the valve.

The present disclosure provides a control mechanism configured to control desufflation flow of an access apparatus. The control mechanism includes an elongated valve that is in fluid communication with the longitudinal passage. The valve includes one or more openings that are operably disposed along a length thereof. The control mechanism includes a selectively movable mechanical interface that is operably coupled to the valve and in operative communication with the opening. The selectively movable mechanical interface is configured to alter desufflation flow when it is moved along a length of the valve.

The present disclosure also provides an access apparatus for use in surgical procedures. The access apparatus includes an access member that defines a longitudinal axis and has a longitudinal passage. The longitudinal passage adapted to permit passage a surgical instrument utilized in performing a surgical procedure. A housing includes a proximal end that defines an opening in communication with the longitudinal passage of the access member to permit passage of the surgical instrument. A zero-closure valve is disposed within the longitudinal passage and is configured to provide a substantially fluid-tight seal in the absence of the surgical instrument inserted therethrough. A control mechanism is configured to control desufflation flow of the access apparatus. The control mechanism includes a valve in fluid communication with the longitudinal passage. The valve includes one or more openings and a selectively movable mechanical interface operably coupled thereto. The selectively movable mechanical interface is configured to alter desufflation flow when it is moved with respect to the valve and across the one or more openings.

### BRIEF DESCRIPTION OF THE DRAWINGS

Various embodiments of the present disclosure are described herein with reference to the drawings wherein:

FIG. 1 is a side cross-sectional view of an access apparatus including a desufflation control mechanism according to an embodiment of the present disclosure;

FIGS. 2A-2C are side, partial cross-sectional views illustrating a sequence of operation of the desufflation control mechanism depicted in FIG. 1; and

FIGS. 3A-3C are side, partial cross-sectional views illustrating a sequence of operation of a desufflation control mechanism according to an alternate embodiment of the present disclosure.

### DETAILED DESCRIPTION

The access apparatus of the present disclosure provides a substantially fluid-tight seal between a body cavity of a patient and the outside atmosphere. The access apparatus of the present disclosure is configured to receive surgical instruments of varying diameters. Included among the various procedures contemplated by the present disclosure are endoscopic, laparoscopic, etc.

The access apparatus of the present disclosure contemplates the introduction of various types of instrumentation during the particular procedure. Examples of instrumentation include, but are not limited to, clip appliers, graspers, dissectors, retractors, staplers, laser probes, photographic devices, endoscopes and laparoscopes, tubes, anchors, anchor drives, etc. Such instruments will collectively be referred to as "instruments" or "instrumentation" or "surgical objects."

In the following description, as is traditional, the term "proximal" refers to the portion of the device closer to the operator while the term "distal" refers to the portion of the device farther from the operator.

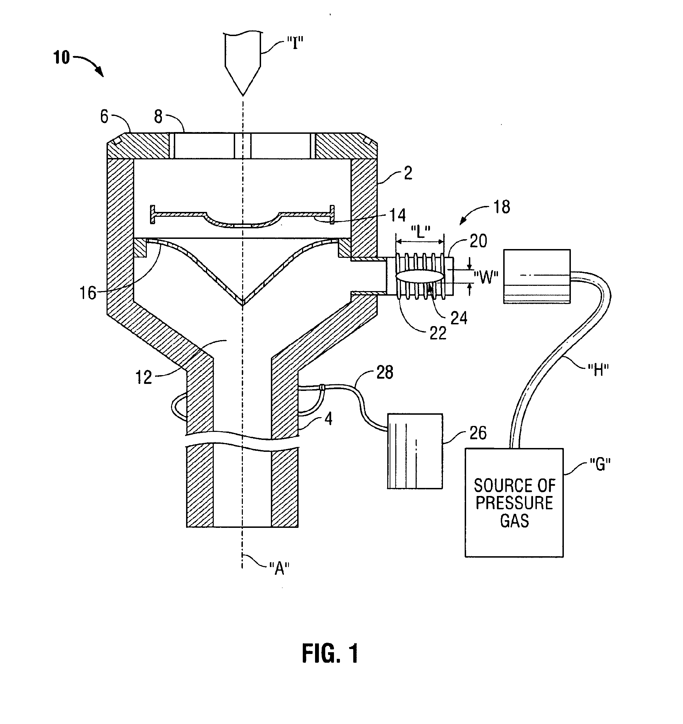

With reference to FIGS. 1-2C, and initially with reference to FIG. 1 an access apparatus designated 10 for use in surgical procedures is shown. Apparatus 10 and operative components associated therewith may be formed from any suitable material, e.g., a biocompatible material. Access apparatus 10 defines a longitudinal axis "A" and includes a housing 2 and an access member 4. A proximal end 6 of housing 2 includes an opening 8 and access member 4 defines a longitudinal passageway 12. Opening 8 and longitudinal passageway 12 are generally aligned with respect to the longitudinal axis "A" to permit passage of surgical objects such as instruments "I" utilized in connection with the procedure.

In the illustrated embodiment, the access apparatus 10 includes one or more instrument seals 14 disposed within housing 2 and adjacent longitudinal passageway 12. Instrument seal 14 is configured to create a substantially fluid-tight seal around an instrument "I" introduced through the instrument seal 14. One suitable instrument seal is disclosed in commonly assigned U.S. Patent No. 6,702,787 to Racenet, the entire contents of which disclosure is incorporated by reference herein.

A duck bill or zero-closure valve 16 is disposed within longitudinal passageway 12 and in mechanical cooperation with housing 2. Zero-closure valve 16 tapers distally and inwardly to a sealed configuration as shown in FIG. 1. As such, zero-closure valve 16 is configured to provide a substantially fluid-tight seal in the absence of a surgical instrument "I" inserted therethrough, as is conventional in the art.

Access apparatus 10 is adapted to connect to a source of pressurized gas "G" (FIG. 1) via one or more suitable interfaces. In the illustrated embodiment, a hose "H" connects access apparatus 10 to the source of pressurized gas "G." More particularly, a distal end of the hose "H" is operably coupled to the source of pressurized gas "G" and a proximal end of the hose "H" operably couples to the access apparatus 10. More particularly, the proximal end of the hose "H" operably couples to a desufflation control mechanism 18 via one or more suitable coupling methods, e.g., a Luer type fitting or the like.

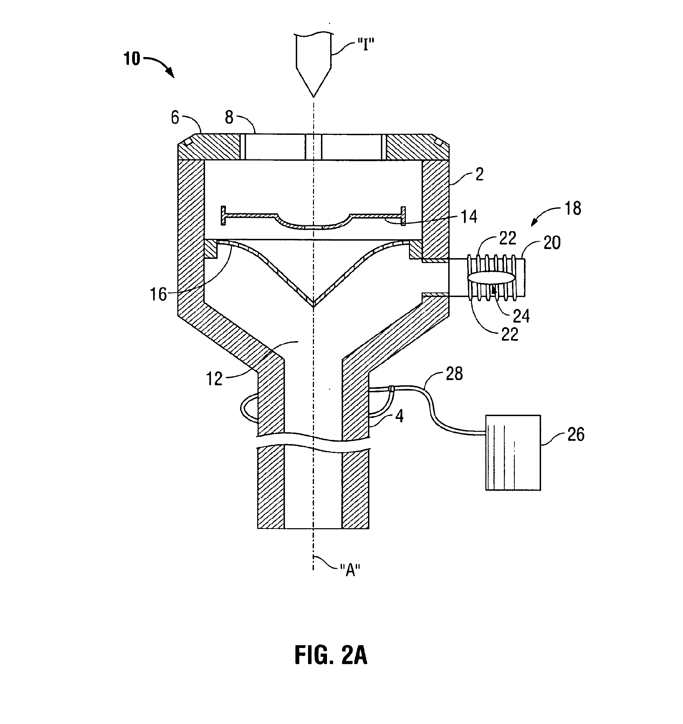

With reference to FIGS. 2A-2C, a desufflation control mechanism 18 (control mechanism 18) according to an embodiment of the present disclosure is illustrated. Control mechanism 18 operably couples to the access apparatus 10 and is configured to control desufflation flow of the access apparatus 10 during a surgical procedure. To this end, the control mechanism 18 includes a valve 20 that is in fluid communication with the longitudinal passage 12 and a selectively movable mechanical interface 26.

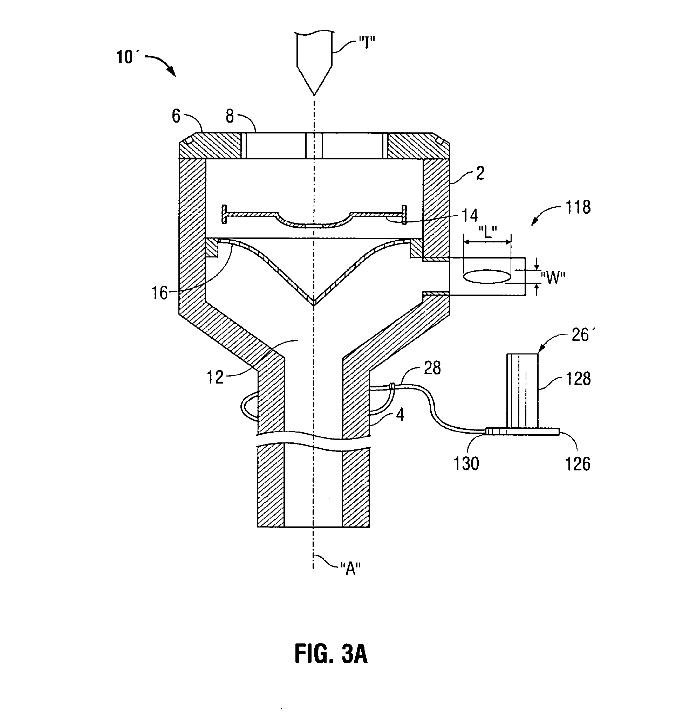

Valve 20 may have any suitable shape. In the illustrated embodiment, valve 20 includes a generally elongated tubular configuration of suitable proportion. Valve 20 may be operably secured to the access apparatus 10 via one or more suitable securement methods, e.g., press fit, friction fit, bayonet fit, integrally formed or ultrasonically/thermally welded etc. In the illustrated embodiment, valve 20 is monolithically formed with the access apparatus 10. Valve 20 is proportioned and configured such that the hose "H" may be removably secured to the access apparatus 10. In the embodiment illustrated in FIGS. 2A-2C, a portion of the valve 20 includes one or more threads 22. The threads 22 provide what is typically referred to in the art as a "Luer-Lok" configuration. Alternatively, valve 20 may not include threads 20; this configuration of valve 20 is typically referred to in the art as a "Luer-Slip" configuration, see FIGS. 3A-3C, for example. Either of these Luer configurations facilitates coupling the proximal end of the hose "H" to the valve 20 in a simple and easy manner.

One or more openings 24 of suitable proportion are operably disposed along a length of the valve 20. In the illustrated embodiment, one opening 24 is operably disposed along a length of one side of the valve 20. In certain embodiments, two openings 24 may be operably disposed on opposite sides of the valve 20. Opening 24 may include any suitable shape. More particularly, opening 24 may include a shape that is selected from the group consisting of circular, oblong, elliptical and square. In the illustrated embodiment, opening 24 includes a generally oblong shape defined by a length "L" and width "W" (FIG. 1). Desufflation flow is controlled by exposing a desired length "L" and "W" of the opening 24. More particularly, desufflation flow is directly related to the exposed area of the opening 24. That is, the more that opening 24 is exposed the greater the amount of desufflation flow.

To control desufflation flow control mechanism 18 includes a selectively movable mechanical interface 26 that is operably coupled to the access apparatus 10. More particularly, selectively movable mechanical interface 26 is operably coupled to the valve 20 and in operative communication with the opening 24. The selectively movable mechanical interface 26 is configured to control and/or alter desufflation flow when it is moved along a length of the valve 20 and across opening 24. Selectively movable mechanical interface 26 is proportioned and shaped to substantially, if not fully, cover the opening 24 when desufflation flow is not required through the opening 24.

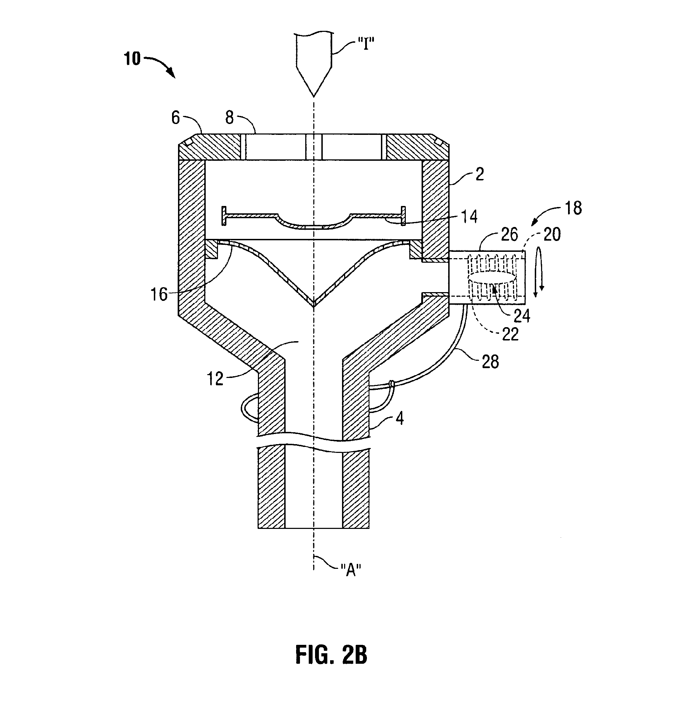

In the embodiment illustrated in FIGS. 2A-2C, selectively movable mechanical interface 26 is in the form of a cap 26 that is movably coupled to the valve 20. More particularly, cap 26 includes a suitable amount of corresponding slots (not explicitly shown) that are configured to rotatably engage the threads 22 that are disposed on the valve 20. This configuration of threads 22 and slots allows the cap 26 to be rotated or "screwed" onto the valve 20. Cap 26 is configured to turn, e.g., in a clockwise and counter clockwise direction, when it is coupled to the valve 20 such that as the cap 26 is turned, the cap 26 moves across a length of the valve 20 and the opening 24. In the illustrated embodiment, clockwise rotation of the cap 26 causes the cap 26 to move distally across the valve 20 and the opening 24, while counter clockwise rotation causes the cap 26 to move proximally across the valve 20 and the opening 24. As can be appreciated, the cap 26 and valve 20 may be configured such that clockwise rotation of the cap 26 causes the cap 26 to move proximally across the valve 20 and the opening 24, while counter clockwise rotation causes the cap 26 to move distally across the valve 20 the opening 24. In accordance with the present disclosure, desufflation flow may be adjusted/controlled by loosening (rotating the cap 26 in a counter clockwise rotation) or by tightening (rotating the cap 26 in a clockwise rotation) to cover and/or expose the opening 24, respectively. In certain embodiments, when the cap 26 is in a distal most position on the valve 20, the opening 24 is fully covered (FIG. 2B), and when the cap 26 is in a proximal most position (or not coupled to the valve 20 (FIG. 2A)), the opening is fully exposed.

In certain embodiments, an exterior of the cap 26 may be textured, e.g., knurled, to facilitate turning the cap 26.

In the illustrated embodiment, cap 26 operably couples to the access apparatus 10 via one or more suitable devices. More particularly, a tether in the form of a cord 28 (made from a suitable material) operably couples the cap 26 to the access apparatus 10 via one or more suitable coupling methods. For example, and as illustrated in FIGS. 2A-2C, cord 28 is coupled to the cap 26 via one or more suitable adhesives, e.g., a heat cured epoxy, and is tied and/or looped around the access apparatus 10 adjacent the access member 4 via one or more types of knot configurations. Coupling the cap 26 to the access apparatus 10 in this manner diminishes the likelihood of misplacing or loosing the cap 26 when the cap 26 is not coupled to the valve 20. In certain instances, the knot and/or portion of the loop may be held in place with respect to the access member 4 via an intent or detent configuration that is operably associated with the access member 4 and/or access apparatus 10.

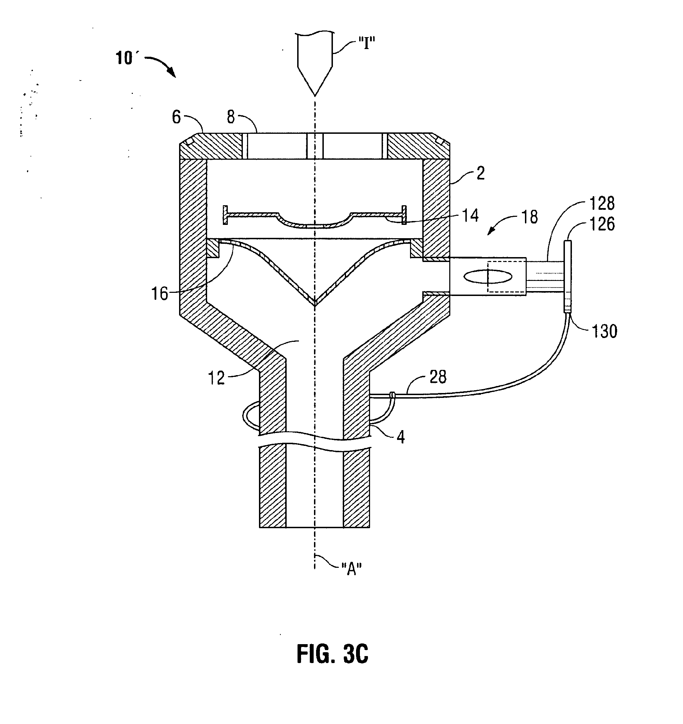

With reference to FIGS. 3A and 3C, an alternate embodiment of the control mechanism 18 is shown designated 118. Control mechanism 118 is substantially similar to control mechanism 18 and, so as not to obscure the present disclosure with redundant information, only those features that are unique to the control mechanism 118 with respect to an access apparatus 10' will be described in further detail.

A selectively movable mechanical interface 26' is in the form of a generally elongated plug or stopper 126 that is made from a suitable material. In one particular embodiment, plug 126 is made from an elastomeric material, e.g., rubber. Plug 126 is proportioned and dimensioned to movably reside in the valve 20 such that the plug 126 may be "pulled" or "pushed" therein or removed therefrom. To this end, plug 126 includes a distal body portion 128 (FIG. 3A) that is configured to movably reside within the valve 20 and a proximal flange or head portion 130 that is configured to contact a portion of the valve 20 when the plug is "pushed" to a predetermined position within the valve 20 such that the opening 24 is fully closed or "blocked-off." Flange 130 provides a surface for a user to grab. Moreover, the flange 130 prevents the plug 126 from being fully wedged or "pushed" into the valve 24.

Plug 126 (or portion thereof, e.g., distal body portion 128) may be coated with or made from a lubricious material to facilitate "pushing" and/or "pulling" the plug 126 with respect to the valve 20 when the distal portion is inserted into the valve 20. One type of lubricious material that the plug 126 may be coated with or made from is tetrafluoroethylene, commonly referred to in the art as and sold under the trademark TEFLON^{®}.

Plug 126 may be tethered as described above with respect to cap 26.

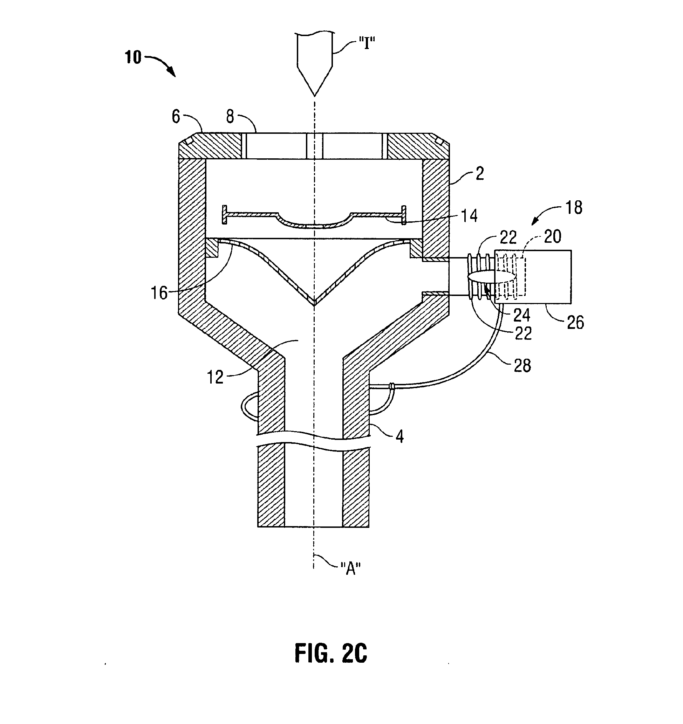

With reference to FIGS. 2A-2C, a method of use of access apparatus 10 with a control mechanism 18 that includes a selectively moveable mechanical interface 26 in the form of a cap 26 described. Access apparatus 10 is securely positioned within an interior of an abdomen through a small incision in tissue. With the cap 26 disconnected from the valve 20 (FIG. 2A), hose "H" is coupled to the valve 20 and the abdominal cavity is insufflated via one or more suitable types of gases, e.g., carbon dioxide, thus creating a pneumoperitoneum. Subsequently, the hose "H" is disconnected from the valve 20 and the cap 26 is coupled to the valve 20 and turned (e.g., in a clockwise direction) until the opening 24 is covered by the cap 26 (FIG. 2B). It should be noted that when the hose "H" is disconnected from the valve 20, a nominal amount of gas escapes from the valve 20. In the instance where the gas within abdominal cavity needs to be cleared and/or evacuated, the cap 26 is turned (e.g., in a counter clockwise direction) such that the cap 26 moves proximally along the valve 20 such that the opening 24, or portion thereof, is exposed (FIG. 2C). With opening 24 fully or partially exposed gas is allowed to flow therethrough until the desired amount of gas is cleared and/or evacuated from the abdominal cavity.

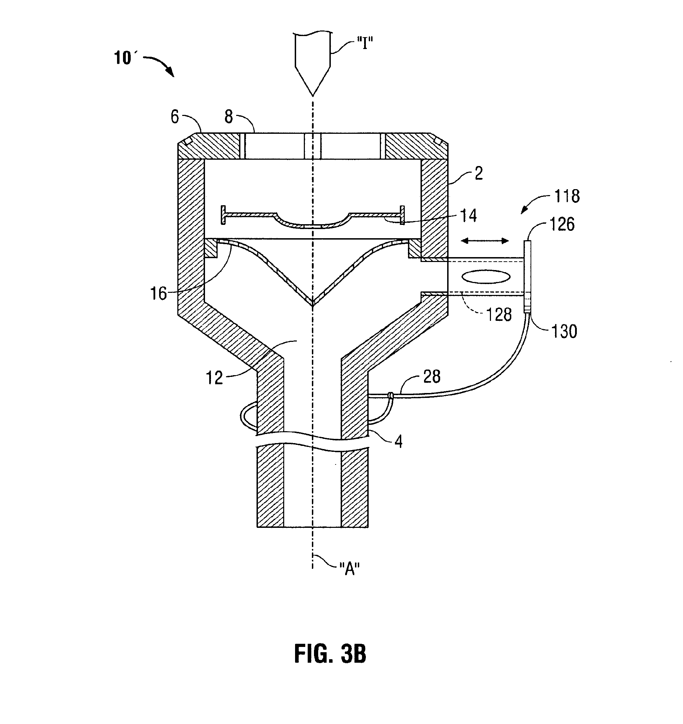

With reference to FIGS. 3A-3C, a method of use of access apparatus 10 with a control mechanism 118 that includes a selectively moveable mechanical interface 26 in the form of a plug 126 described. Access apparatus 10 is securely positioned within an interior of an abdomen through a small incision in tissue. With the plug 126 disconnected from the valve 20 (FIG. 3A), hose "H" is coupled to the valve 20 and the abdominal cavity is insufflated via one or more suitable types of gases, e.g., carbon dioxide, thus creating a pneumoperitoneum. Subsequently, the hose "H" is disconnected from the valve 20 and the plug 126 is coupled to the valve 20 and "pushed" distally across the valve 20 until the opening 24 is closed or "blocked-off" by the plug 126 (FIG. 3B). It should be noted that when the hose "H" is disconnected from the valve 20, a nominal amount of gas escapes from the valve 20. In the instance where the gas within abdominal cavity needs to be released, the plug 126 is "pulled" proximally across the valve 20 such that the opening 24, or portion thereof, is exposed (FIG. 3C). With opening 24 fully or partially exposed gas is allowed to flow therethrough until the desired amount of gas is released from the abdominal cavity.

From the foregoing and with reference to the various figure drawings, those skilled in the art will appreciate that certain modifications can also be made to the present disclosure without departing from the scope of the same. For example, it is contemplated that the cap 26 and valve 20 illustrated in FIGS. 2A-2C may be engageable with each other via a press fit, friction fit or other suitable engagement methods that are suitable for the intended purposes described herein. In this instance, the cap 26 is configured similarly to the plug 126. A distinguishing factor of the cap 26 that is configured to slide across the valve 20 when compared to the plug 126 is that the cap 26 is configured to slide across the valve 20 and over the opening 24.

It is contemplated that, in certain instances, it may prove useful to operably associate either of the control mechanism 18/118 with the distal end the hose "H." For example, control mechanism 18 may be operably coupled to the distal end of the hose "H." In this instance, control mechanism 18 may be configured to control both insufflation and desufflation flow.

It is contemplated that, in certain instances, it may prove useful to have the distal end of the hose "H" and the selectively movably mechanical interfaces, e.g., cap 26/plug 126, removably couple to one another. More particularly, the distal end of the hose "H" may be configured to removably and securely attach to the valve 20 via the cap 26/plug 126 via one or more the previously described coupling methods, e.g., "Luer-Lok."

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:-
1. An access apparatus for use in surgical procedures, comprising:
   an access member defining a longitudinal axis and having a longitudinal passage, the longitudinal passage being adapted to permit passage a surgical instrument utilized in performing a surgical procedure;
   a housing including a proximal end defining an opening in communication with the longitudinal passage of the access member to permit passage of the surgical instrument;
   a zero-closure valve disposed within the longitudinal passage and configured to provide a substantially fluid-tight seal in the absence of the surgical instrument inserted therethrough; and
   a control mechanism operably coupled to the access apparatus and configured to control desufflation flow of the access apparatus, the control mechanism including an elongated valve in fluid communication with the longitudinal passage, the elongated valve including at least one opening operably disposed along a length thereof, the control mechanism includes a selectively movable mechanical interface that is operably coupled to the valve and in operative communication with the at least one opening, the selectively movable mechanical interface configured to alter desufflation flow when it is moved along a length of the valve.
2. An access apparatus according to paragraph 1, wherein the valve is threaded.
3. An access apparatus according to paragraph 1, wherein the selectively movable mechanical interface is tethered to the access apparatus.
4. An access apparatus according to paragraph 1, wherein the selectively movable mechanical interface is a cap.
5. An access apparatus according to paragraph 4, wherein the selectively movable mechanical interface is rotatably coupled to the valve.
6. An access apparatus according to paragraph 1, wherein the selectively movable mechanical interface is a plug.
7. An access apparatus according to paragraph 6, wherein the selectively movable mechanical interface is slidably coupled to the valve and movable therein.
8. An access apparatus according to paragraph 1, wherein the at least one opening includes a shape that is selected from the group consisting of circular, oblong, elliptical and square.
9. An access apparatus according to paragraph 1, wherein the access apparatus is adapted to couple to a source of pressurized gas.
10. A control mechanism configured to control desufflation flow of an access apparatus, comprising:
   an elongated valve in fluid communication with the longitudinal passage, the valve including at least one opening operably disposed along a length thereof, the valve including a selectively movable mechanical interface that is operably coupled to the valve and in operative communication with the at least one opening, the selectively movable mechanical interface configured to alter desufflation flow when it is moved along a length of the valve.
11. A control mechanism according to paragraph 10, wherein the valve is threaded.
12. A control mechanism according to paragraph 10, wherein the selectively movable mechanical interface is tethered to the access apparatus.
13. A control mechanism according to paragraph 10, wherein the selectively movable mechanical interface is a cap.
14. A control mechanism according to paragraph 13, wherein the selectively movable mechanical interface is rotatably coupled to the valve.
15. A control mechanism according to paragraph 10, wherein the selectively movable mechanical interface is a plug.
16. A control mechanism according to paragraph 15, wherein the selectively movable mechanical interface is slidably coupled to the valve.
17. A control mechanism according to paragraph 10, wherein the at least one opening includes a shape that is selected from the group consisting of circular, oblong, elliptical and square.
18. A control mechanism according to paragraph 1, wherein the access apparatus is adapted to couple to a source of pressurized gas.
19. An access apparatus for use in surgical procedures, comprising:
   an access member defining a longitudinal axis and having a longitudinal passage, the longitudinal passage being adapted to permit passage a surgical instrument utilized in performing a surgical procedure;
   a housing including a proximal end defining an opening in communication with the longitudinal passage of the access member to permit passage of the surgical instrument;
   a zero-closure valve operably associated with the access member and configured to provide a substantially fluid-tight seal in the absence of the surgical instrument inserted therethrough; and
   a control mechanism configured to control desufflation flow of the access apparatus, the control mechanism including a valve in fluid communication with the longitudinal passage, the valve including at least one opening and a selectively movable mechanical interface operably coupled thereto, the selectively movable mechanical interface configured to alter desufflation flow when it is moved with respect to the valve and across the at least one opening.

## Claims

1. An access apparatus for use in surgical procedures, comprising:
an access member defining a longitudinal axis and having a longitudinal passage, the longitudinal passage being adapted to permit passage a surgical instrument utilized in performing a surgical procedure;
a housing including a proximal end defining an opening in communication with the longitudinal passage of the access member to permit passage of the surgical instrument;
a zero-closure valve disposed within the longitudinal passage and configured to provide a substantially fluid-tight seal in the absence of the surgical instrument inserted therethrough; and
a control mechanism operably coupled to the access apparatus and configured to control desufflation flow of the access apparatus, the control mechanism including an elongated valve in fluid communication with the longitudinal passage, the elongated valve including at least one opening operably disposed along a length thereof, the control mechanism includes a selectively movable mechanical interface that is operably coupled to the valve and in operative communication with the at least one opening, the selectively movable mechanical interface configured to alter desufflation flow when it is moved along a length of the valve.

2. An access apparatus according to claim 1, wherein the valve is threaded.

3. An access apparatus according to claim 1 or claim 2, wherein the selectively movable mechanical interface is tethered to the access apparatus.

4. An access apparatus according to any preceding claim, wherein the selectively movable mechanical interface is a cap, preferably wherein the selectively movable mechanical interface is rotatably coupled to the valve.

5. An access apparatus according to any preceding claim, wherein the selectively movable mechanical interface is a plug, preferably wherein the selectively movable mechanical interface is slidably coupled to the valve and movable therein.

6. An access apparatus according to any preceding claim, wherein the at least one opening includes a shape that is selected from the group consisting of circular, oblong, elliptical and square.

7. An access apparatus according to any preceding claim, wherein the access apparatus is adapted to couple to a source of pressurized gas.

8. A control mechanism configured to control desufflation flow of an access apparatus, comprising:
an elongated valve in fluid communication with the longitudinal passage, the valve including at least one opening operably disposed along a length thereof, the valve including a selectively movable mechanical interface that is operably coupled to the valve and in operative communication with the at least one opening, the selectively movable mechanical interface configured to alter desufflation flow when it is moved along a length of the valve.

9. A control mechanism according to claim 8, wherein the valve is threaded.

10. A control mechanism according to claim 8 or claim 9, wherein the selectively movable mechanical interface is tethered to the access apparatus.

11. A control mechanism according to any of claims 8 to 10, wherein the selectively movable mechanical interface is a cap, preferably wherein the selectively movable mechanical interface is rotatably coupled to the valve.

12. A control mechanism according to any of claims 8 to 11, wherein the selectively movable mechanical interface is a plug, preferably wherein the selectively movable mechanical interface is slidably coupled to the valve.

13. A control mechanism according to any of claims 8 to 12, wherein the at least one opening includes a shape that is selected from the group consisting of circular, oblong, elliptical and square.

14. A control mechanism according to any preceding claim, wherein the access apparatus is adapted to couple to a source of pressurized gas.

15. An access apparatus for use in surgical procedures, comprising:
an access member defining a longitudinal axis and having a longitudinal passage, the longitudinal passage being adapted to permit passage a surgical instrument utilized in performing a surgical procedure;
a housing including a proximal end defining an opening in communication with the longitudinal passage of the access member to permit passage of the surgical instrument;
a zero-closure valve operably associated with the access member and configured to provide a substantially fluid-tight seal in the absence of the surgical instrument inserted therethrough; and
a control mechanism configured to control desufflation flow of the access apparatus, the control mechanism including a valve in fluid communication with the longitudinal passage, the valve including at least one opening and a selectively movable mechanical interface operably coupled thereto, the selectively movable mechanical interface configured to alter desufflation flow when it is moved with respect to the valve and across the at least one opening.
